# FASCICULE DE BREVET EUROPEEN

(11) **EP 3 315 104 B1**
(45) Date de publication et mention de la délivrance du brevet: **01.05.2019**
(21) Numéro de dépôt: 17196787.0
(22) Date de dépôt: 17.10.2017
(51) Int. Cl.: A61F 5/448, A61F 5/449

(54) **DISPOSITIF MEDICAL POUR STOMIE**
MEDIZINISCHE VORRICHTUNG FÜR STOMA
MEDICAL DEVICE FOR STOMA

(30) Priorité: 26.10.2016 FR 1660396
(43) Date de publication de la demande: 02.05.2018
(73) Titulaire: Vlamynck, Elodie, 80120 Fort Mahon Plage (FR); Gagnoux, Jérôme, 02100 Saint-Quentin (FR)
(72) Inventeur: Vlamynck, Elodie, 80000 Amiens (FR); Gagnoux, Jérôme, 02100 Saint Quentin (FR)
(74) Mandataire: Cabinet Rifflart Vandenbossche

(56) Documents cités:
- WO-A1-2015/171173
- CN-U- 201 743 812
- CN-Y- 2 877 637
- US-A- 2 505 664
- US-A- 2 527 321
- US-A1- 2016 045 359

## Description

### Domaine technique

La présente invention se rapporte au domaine des appareillages de recueil pour stomie. L'invention vise tout particulièrement à faciliter la mise en place et le maintien d'un tel appareillage de recueil sur un patient ayant subi une telle stomie.

### Etat de la technique

Un appareillage de recueil pour stomie permet de recueillir, de stocker et d'évacuer les selles voire les urines dans le cadre d'une stomie. Une stomie consiste à aboucher une portion de l'intestin à la peau d'une personne, l'appareillage de recueil étant positionné au niveau de cet abouchement à la peau pour être raccordé à l'intestin.

L'appareillage de recueil est choisi en fonction du type de stomie et de sa localisation, ainsi que du mode de vie de la personne, généralement avec les conseils d'un infirmier ou d'une infirmière stomathérapeute.

L'appareillage de recueil se compose d'une poche de recueil et d'un support qui permet de fixer la poche sur l'abdomen. Il existe deux types d'appareillages de recueil, ceux constitués d'une seule pièce et ceux constitués de deux pièces.

Dans le cas d'un appareillage de recueil en une pièce, le support et la poche sont inséparables l'un de l'autre, ledit support étant directement positionné sur la peau en regard de la stomie.

Dans le cas d'un appareillage de recueil en deux pièces, la poche et le support sont indépendants ; le support est d'abord positionné sur la peau en regard de la stomie puis la poche est assemblée avec le support. Cette seconde conception présente pour avantage de pouvoir remplacer uniquement la poche et de faciliter son retrait pour la vidanger, le support devant en général être remplacé tous les deux ou trois jours selon son état.

Il est d'usage de positionner une ceinture abdominale afin de maintenir la poche de l'appareillage de recueil en une pièce ou en deux pièces. Cela nécessite la réalisation d'une ouverture sur la ceinture abdominale dans la zone correspondant à la stomie afin de pouvoir placer la poche et le support chacun d'un côté de l'ouverture. La poche est positionnée du côté externe de la ceinture abdominale pour permettre son augmentation de volume durant son remplissage ; cela permet en outre son retrait du support dans le cas d'un appareillage de recueil en deux pièces. Le support est positionné du côté interne de la ceinture abdominale qui assure un maintien en appui contre l'abdomen.

Certaines ceintures abdominales sont munies d'une ouverture préexistante. Ce type de ceinture est cependant peu pratique puisque l'ouverture doit correspondre précisément à la position de la stomie une fois la ceinture convenablement positionnée autour du buste. Le positionnement de l'ouverture en regard du support et de la stomie implique donc généralement le port de la ceinture de manière inconfortable.

C'est pourquoi il est préférable de réaliser une découpe dans la ceinture abdominale une fois celle-ci convenablement positionnée autour du buste avec de créer cette ouverture en correspondance avec la stomie.

Certaines ceintures abdominales disposent d'un plastron qui peut être découpé. La réalisation de la découpe nécessite cependant de renvoyer la ceinture chez le fabricant pour finaliser l'ouverture en effectuant un point d'arrêt autour de la découpe afin d'éviter le démaillage du plastron. Ce point d'arrêt autour de la découpe peut également être effectué par un appareilleur, par exemple un orthésiste ou un pharmacien. La personne ne peut donc disposer immédiatement de sa ceinture, ce qui peut être problématique lorsqu'il convient de remplacer rapidement une ceinture souillée suite à un incident se produisant lors du retrait de la poche.

D'autres ceintures abdominales disposent de deux plastrons amovibles, l'un utilisé temporairement avec un risque de démaillage au niveau de la découpe durant son utilisation et l'autre renvoyé chez le fabricant pour effectuer un point d'arrêt autour de la découpe et éviter un tel démaillage. Cela augmente le coût de la ceinture.

D'autres ceintures abdominales disposent d'un plastron confectionné dans un textile indémaillable, ce qui permet de découper le plastron et d'utiliser la ceinture immédiatement.

Il est connu des dispositifs médicaux pour stomie conçus pour être rapportés sur des ceintures abdominales et pour recevoir un appareil de recueil pour stomie. On citera les documents CN2877637Y, CN201743812U et US2505664A.

Dans le document CN2877637Y, le dispositif médical pour stomie comprend une plaque sur les extrémités latérales de laquelle sont fixées les extrémités latérales de la ceinture. La plaque comprend une portion cylindrique avec une ouverture centrale débouchant sur la plaque et un contour externe muni d'une gorge destinée à recevoir un bord cylindrique d'une poche pour le recueil, cette poche de recueil communiquant avec l'ouverture centrale.

Dans les documents CN201743812U et US2505664A, le dispositif médical pour stomie se présente sous la forme d'une pièce annulaire munie d'une ouverture centrale et d'une portion épaulée sur le contour externe de laquelle est agencée une gorge permettant la réception d'un bord périphérique d'une ouverture mise en oeuvre sur une ceinture abdominale. Dans le document CN201743812U, cette gorge est également destinée à recevoir un bord cylindrique d'une poche pour le recueil, la poche de recueil communiquant avec l'ouverture centrale. Dans le document US2505664A, l'ouverture centrale est configurée pour recevoir l'extrémité d'une poche de recueil, un anneau interne étant configuré pour s'insérer dans l'ouverture centrale, depuis une face arrière de la pièce annulaire, de sorte à pincer l'extrémité de la poche de recueil entre ladite pièce annulaire et ledit anneau interne, pour la fixation de la poche de recueil avec le dispositif médical.

### Résumé de l'invention

La présente invention a pour objectif de permettre l'utilisation d'une ceinture abdominale pour stomie dès sa découpe, en palliant les risques de démaillage au niveau de cette découpe. Un autre objectif est de garantir le maintien convenable de l'appareil de recueil au moyen de la ceinture.

A cet effet, l'invention concerne un dispositif médical pour stomie qui est configuré pour être rapporté sur une ceinture abdominale, au niveau d'une découpe réalisée sur ladite ceinture, et pour recevoir un appareillage de recueil pour stomie en une pièce ou en deux pièces.

Selon l'invention, le dispositif médical comprend un anneau comportant un contour interne, un contour externe et deux faces, une face interne disposée du côté du support appliqué en regard de la stomie et une face externe disposée du côté de la poche.

Le contour interne définit une ouverture centrale configurée pour réceptionner l'appareillage de recueil. L'ouverture centrale permet soit le passage de la poche vide de l'intérieur vers l'extérieur de la ceinture abdominale, dans le cas d'un appareillage de recueil en une pièce, soit l'assemblage entre le support et la poche disposés respectivement contre les faces interne et externe de l'anneau, dans le cas d'un appareillage de recueil en deux pièces.

En outre, l'anneau comprend une gorge qui s'étend radialement vers l'intérieur depuis son contour externe, ladite gorge étant configurée pour réceptionner un bord périphérique d'une ouverture mise en oeuvre sur la ceinture abdominale par découpage de celle-ci.

En outre, l'anneau comprend au moins une lumière débouchant sur les deux faces, ladite au moins une lumière traversant la gorge. De préférence, quatre lumières sont uniformément réparties autour de l'anneau.

Ainsi, la présence de l'anneau permet avantageusement d'éviter un contact direct de l'appareillage de recueil avec l'ouverture sur la ceinture abdominale, ce qui permet de manipuler l'appareillage de recueil sans risque de démaillage un niveau de l'ouverture sur la ceinture abdominale durant le passage de la poche dans ladite ouverture voire durant l'emboîtement ou le retrait de la poche vis-à-vis de son support. Il est donc possible de réaliser une découpe sur la ceinture abdominale et de l'utiliser aussitôt après avoir positionné le dispositif médical, sans risque de démaillage. Ces lumières permettent de fixer le dispositif médical sur la ceinture abdominale, notamment au moyen de coutures, de pièces de rivetage ou de pièces de pression, ce qui empêche tout risque de déplacement de l'anneau sur la ceinture abdominale et tout risque de démaillage au niveau de la découpe sur la ceinture abdominale. Ainsi, le dispositif médical optimise le maintien de l'appareil de recueil au moyen de la ceinture abdominale et la durée de vie de la ceinture abdominale.

Le terme anneau couvre la forme d'anneau en tant que telle, c'est-à-dire avec une forme circulaire, mais aussi des formes plus ou moins circulaires, par exemple une forme elliptique, voire tout autre forme qui serait adaptée à une forme de découpe réalisée sur la ceinture abdominale et à la forme des moyens d'assemblage entre le support et la poche de l'appareillage de recueil.

Selon une réalisation du dispositif médical, l'anneau est plat. On pourrait toutefois prévoir des formes différentes de ses deux faces, par exemple une face interne plus ou moins convexe adaptée à la forme du support et une face externe plane adaptée à la forme de la poche.

Selon le dispositif médical, celui-ci est réalisé dans une matière offrant une souplesse. De préférence, le dispositif médical est réalisé en silicone de grade médical. Cela permet d'épouser convenablement la forme de l'abdomen en fonction de la position de la stomie. En outre, cette souplesse, associée à la fixation entre la ceinture et le dispositif médical grâce à la présence des lumières, permet de déformer l'anneau dans des proportions équivalentes à celles de la ceinture abdominale, ce qui améliore encore mieux le confort de l'utilisateur et le maintien de l'appareil de recueil au moyen de la ceinture abdominale.

On pourrait envisager l'utilisation d'autres matières offrant plus ou moins de souplesse, sans sortir du cadre de l'invention. La présence du dispositif médical sur la ceinture abdominale et le choix d'une matière siliconée à grade médical facilitent en outre le nettoyage lors du remplacement de l'appareillage de recueil.

Selon le dispositif médical, la gorge comprend une largeur comprise entre 1,5 mm et 2,5 mm, de préférence 2 mm. Cela permet la réception de différentes épaisseurs de ceintures abdominales.

Selon le dispositif médical, la gorge comprend une profondeur comprise entre 18 mm et 23 mm, de préférence 21,5 mm. Cela garantit un maintien convenable du bord périphérique de l'ouverture sur la ceinture abdominale à l'intérieur de la gorge en évitant les risques de démaillage.

Selon le dispositif médical, l'anneau comprend un contour interne présentant un diamètre interne compris entre 60 mm et 70 mm, de préférence 65 mm, et un contour externe présentant un diamètre externe compris entre 110 mm et 120 mm, de préférence 115 mm. On pourrait toutefois adapter ces diamètres interne et externe en fonction des dimensions de l'appareillage de recueil, notamment les diamètres des portions d'emboîtement mises en oeuvre sur le support et sur la poche, comme cela apparaîtra dans la suite de la description.

L'invention concerne également un kit de stomie comprenant au moins une ceinture abdominale et un dispositif médical présentant l'une ou l'autre des caractéristiques précitées. Le kit de stomie comprend des moyens de fixation configurés pour fixer le dispositif médical avec le bord périphérique de l'ouverture sur la ceinture abdominale, lesdits moyens de fixation étant mis en place au niveau de l'au moins une lumière sur ledit dispositif médical. De préférence, les moyens de fixation sont de type couture, pièce de rivetage ou pièce de pression.

### Brève description des figures

Les caractéristiques et avantages de l'invention apparaîtront à la lecture de la description suivante s'appuyant sur des figures, parmi lesquelles :
- La figure 1 illustre une vue tridimensionnelle d'un mode de réalisation préféré du dispositif médical objet de l'invention,
- La figure 2 illustre une vue en plan du dispositif médical de la figure 1,
- La figure 3 illustre une vue en coupe du dispositif médical de la figure 2, mis en place sur une ceinture abdominale,
- La figure 4 est une vue partielle agrandie de la figure 3,
- La figure 5 illustre un appareillage de recueil en deux pièces mis en place sur un dispositif médical selon l'invention,
- La figure 6 illustre un support constituant la première pièce de l'appareillage de recueil,
- La figure 7 illustre une poche constituant la deuxième pièce de l'appareillage de recueil.

### Description détaillée

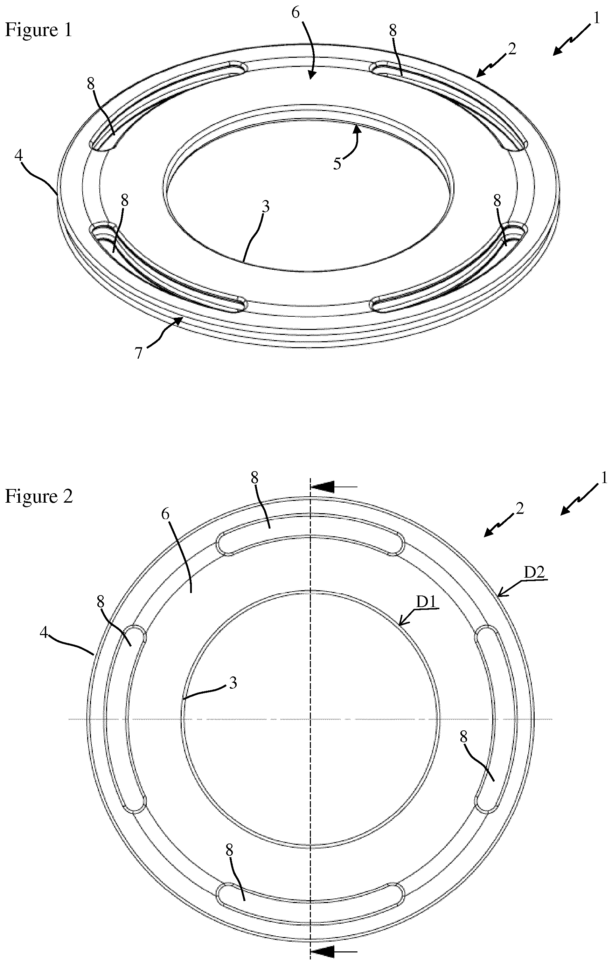

Tel qu'illustré sur les figures 1 à 5, le dispositif médical 1 selon l'invention comprend préférentiellement la forme d'un anneau 2 qui est plat et qui présente un contour interne 3, un contour externe 4, une face interne 5 et une face externe 6. Cette anneau 2 est réalisé dans une matière siliconée ce qui la rend souple afin de pouvoir la déformer et l'adapter au mieux selon la position de la stomie sur l'abdomen de la personne.

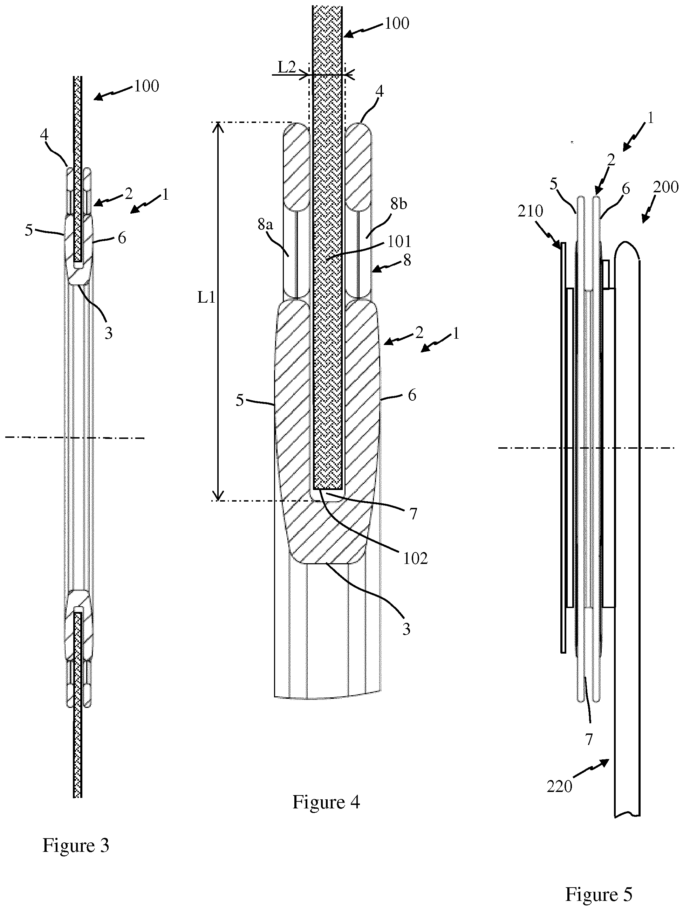

Le dispositif médical 1 comprend une gorge 7 qui, sur l'anneau 2, est préférentiellement circulaire. La gorge 7 s'étend à l'intérieur de l'anneau 2 depuis son contour externe 4. Cette gorge 7 reçoit le bord périphérique 101 d'une ouverture 102 d'une ceinture abdominale 100, comme l'illustrent les figures 3 et 4. La position de l'ouverture 102 sur la ceinture abdominale 100 dépend de la position de la stomie. Cette ouverture 102 est réalisée par découpage de la ceinture abdominale 100.

La gorge 7 est dimensionnée pour que le bord périphérique 101 de l'ouverture 102 pénètre de manière suffisamment importante dans celle-ci, ce qui évite tout risque de démaillage de la ceinture abdominale 100. Pour cela, la gorge 7 comprend une profondeur L1 comprise entre 18 mm et 23 mm, de préférence cette profondeur L1 étant égale à 21,5 mm.

De même, la gorge 7 est dimensionnée pour recevoir plusieurs épaisseurs de ceinture abdominale 100. De préférence, la gorge 7 comprend une largeur L2 comprise entre 1,5 mm et 2,5 mm, de préférence cette largeur L2 étant égale à 2 mm.

Le dispositif médical 1 comprend quatre lumières 8 qui débouchent chacune sur la face interne 5 et sur la face externe 6 de l'anneau 2, ces lumières 8 traversant la gorge 7, comme l'illustrent les figures 1 à 4. Ces lumières 8 ont une forme incurvée, de préférence circulaire, et elles sont uniformément réparties autour de l'anneau 2. Lorsque le dispositif médical 1 est positionné sur une ouverture 102 d'une ceinture abdominale 100, le bord périphérique 101 est positionné entre deux parties 8a, 8b de chaque lumière 8. Cette mise en oeuvre facilite la fixation par couture du dispositif médical 1 avec la ceinture abdominale 100. On pourrait envisager d'autres formes pour les lumières 8, par exemple de simples trous circulaires. De même, on pourrait envisager d'autres moyens de fixation que des coutures, par exemple pièces de rivetage ou des pièces de pression.

Le dispositif médical 1 permet la réception d'un appareillage de recueil 200 qui comprend un support 210 et une poche 220. Le support 210 est positionné du côté de la face interne 5 du dispositif médical 1, côté interne de la ceinture abdominale 100, et la poche 220 est positionnée du côté de la face externe 6 dudit dispositif, côté externe de la ceinture abdominale 100, comme l'illustrent les figures 3 à 5.

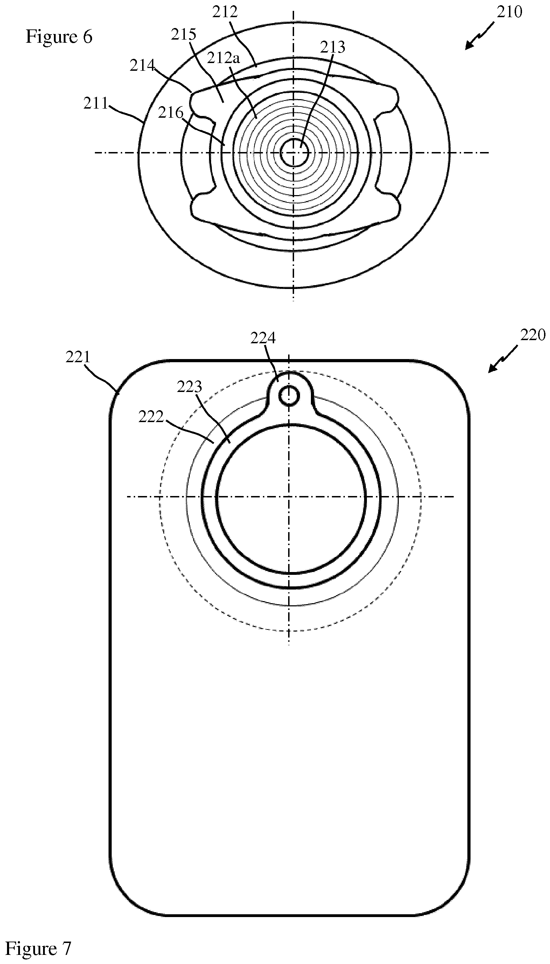

Tel qu'illustré en figure 6, le support 210 comprend une plaque 211 qui est souple et qui se colle sur la peau de la personne. La plaque 211 comporte une gomme 212 dont le centre 212a est muni d'un trou 213 dimensionné en fonction de l'abouchement de la stomie. Le support 210 comprend également une première pièce d'assemblage 214 qui est rigide et qui comporte une base 215 qui prend appui sur la face interne 5 de l'anneau 2 et une portion cylindrique mâle 216 s'étendant dans le sens opposé à la plaque 211 et communiquant avec le trou 213, cette portion cylindrique mâle 216 pénétrant dans l'anneau 2.

Tel qu'illustré en figure 7, la poche 220 comprend une enveloppe souple 221 et une seconde pièce d'assemblage 222 qui est rigide et qui communique avec l'intérieur de cette enveloppe souple 221. La seconde pièce d'assemblage 222 comprend une portion cylindrique femelle 223 configurée pour recevoir par emboitement la portion cylindrique mâle 216. La seconde pièce d'assemblage 222 comprend également une languette 224 qui facilite la manipulation pour le déboîtement de la poche 220 vis-à-vis du support 210.

Le contour interne 3 de l'anneau 2 définit une ouverture centrale 9 qui permet le passage de la portion cylindrique mâle 216 pour venir l'emboîter dans la portion cylindrique femelle 223 lors de l'assemblage de la poche 220 avec le support 210. Le contour interne 3 de l'anneau 2 présente un diamètre interne D1 compris entre 60 mm et 70 mm, de préférence égal à 65 mm. De même, le contour externe 4 présente un diamètre externe D2 compris entre 110 mm et 120 mm, de préférence égal à 115 mm.

La description détaillée qui précède d'un mode de réalisation particulier de l'invention n'a aucun caractère limitatif. Bien au contraire, elle a pour objectif d'ôter toute éventuelle imprécision quant à sa portée. Ainsi, de nombreuses variantes pourront être envisagées dans le cadre de l'invention, notamment quant à la conception de l'anneau 2.

Par exemple, lorsque le support 210 est semi convexe ou convexe, la plaque 211 qui adhère à la peau présente une convexité plus ou moins prononcées permettant de faire ressortir la stomie. Dans ce cas, la face interne 5 de l'anneau 2 pourra présenter également une convexité adaptée pour recevoir convenablement le support 210. L'invention concerne également un kit de stomie qui comprend un dispositif médical 1, une ceinture abdominale 100 et un appareillage de recueil 200.

La description qui précède se réfère à un appareillage de recueil 200 en deux pièces selon lequel le support 210 et la poche 220 sont séparables l'un de l'autre. Le dispositif médical 1 selon l'invention trouve également son application avec un appareillage de recueil 200 en une pièce selon lequel le support 210 et la poche 220 sont inséparables. Dans ce cas, une fois le support 210 appliqué au niveau de la stomie sur la peau de la personne, la poche 220 vide est insérée au travers l'ouverture centrale 9 de l'anneau 2 pour être disposée du côté externe de la ceinture abdominale 100, ladite ceinture abdominale 100 permettant de maintenir le support contre l'abdomen.

## Revendications

1. Dispositif médical (1) pour stomie, comprenant un anneau (2) comportant un contour interne (3), un contour externe (4) et deux faces (5, 6), le contour interne définissant une ouverture centrale (9) configurée pour réceptionner un appareillage de recueil (200) pour stomie, ledit anneau comprenant une gorge (7) s'étendant radialement depuis son contour externe, ladite gorge étant configurée pour réceptionner un bord périphérique (101) d'une ouverture (102) mise en oeuvre sur une ceinture abdominale (100), **caractérisé en ce que** l'anneau (2) comprend au moins une lumière (8) débouchant sur les deux faces (5, 6), ladite au moins une lumière traversant la gorge (7).

2. Dispositif médical (1) selon la revendication 1, dans laquelle l'anneau (2) est plat.

3. Dispositif médical (1) selon l'une des revendications précédentes, lequel est réalisé dans une matière offrant une souplesse.

4. Dispositif médical (1) selon la revendication 3, lequel est réalisé en silicone médical.

5. Dispositif médical (1) selon l'une des revendications précédentes, dans lequel quatre lumières (8) sont uniformément réparties autour de l'anneau (2).

6. Dispositif médical (1) selon l'une des revendications précédentes, dans lequel la gorge (7) comprend une largeur (L2) comprise entre 1,5 mm et 2,5 mm, de préférence 2 mm.

7. Dispositif médical (1) selon l'une des revendications précédentes, dans lequel la gorge (2) comprend une profondeur (L1) comprise entre 18 mm et 23 mm, de préférence 21,5 mm.

8. Dispositif médical (1) selon l'une des revendications précédentes, dans lequel l'anneau (2) comprend un contour interne (3) présentant un diamètre interne (D1) compris entre 60 mm et 70 mm, de préférence 65 mm, et un contour externe (4) présentant un diamètre externe (D2) compris entre 110 mm et 120 mm, de préférence 115 mm.

9. Kit de stomie comprenant au moins une ceinture abdominale (100) et un dispositif médical (1) selon l'une des revendications précédentes.

10. Kit de stomie selon la revendication 9, lequel comprend des moyens de fixation configurés pour fixer le dispositif médical (1) avec le bord périphérique (101) de l'ouverture (102) sur la ceinture abdominale (100), lesdits moyens de fixation étant mis en place au niveau de l'au moins une lumière (8) sur ledit dispositif médical (1).

11. Kit de stomie selon la revendication 10, dans lequel les moyens de fixation sont de type couture, pièce de rivetage ou pièce de pression.

## Patentansprüche

1. Medizinische Stomavorrichtung (1), umfassend einen Ring (2), der eine Innenkontur (3), eine Außenkontur (4) und zwei Flächen (5, 6) umfasst, wobei die Innenkontur eine mittige Öffnung (9) definiert, die dafür ausgebildet ist, eine Stomaauffangeinrichtung (200) aufzunehmen, wobei der Ring eine Kehle (7) umfasst, die sich radial von seiner Außenkontur erstreckt, wobei die Kehle dafür ausgebildet ist, einen Umfangsrand (101) einer Öffnung (102) aufzunehmen, die an einem Bauchgurt (100) umgesetzt ist, **dadurch gekennzeichnet, dass** der Ring (2) mindestens einen Durchbruch (8) umfasst, der an den zwei Flächen (5, 6) mündet, wobei der mindestens eine Durchbruch durch die Kehle (7) hindurchgeht.

2. Medizinische Vorrichtung (1) nach Anspruch 1, wobei der Ring (2) flach ist.

3. Medizinische Vorrichtung (1) nach einem der vorstehenden Ansprüche, die in einem Material ausgeführt ist, das eine Nachgiebigkeit bietet.

4. Medizinische Vorrichtung (1) nach Anspruch 3, die aus medizinischem Silikon ausgeführt ist.

5. Medizinische Vorrichtung (1) nach einem der vorstehenden Ansprüche, wobei vier Durchbrüche (8) gleichmäßig um den Ring (2) herum verteilt sind.

6. Medizinische Vorrichtung (1) nach einem der vorstehenden Ansprüche, wobei die Kehle (7) eine Breite (L2) im Bereich zwischen 1,5 mm und 2,5 mm, vorzugsweise 2 mm, umfasst.

7. Medizinische Vorrichtung (1) nach einem der vorstehenden Ansprüche, wobei die Kehle (2) eine Tiefe (L1) im Bereich zwischen 18 mm und 23 mm, vorzugsweise 21,5 mm, umfasst.

8. Medizinische Vorrichtung (1) nach einem der vorstehenden Ansprüche, wobei der Ring (2) eine Innenkontur (3), die einen Innendurchmesser (D1) im Bereich zwischen 60 mm und 70 mm, vorzugsweise 65 mm, aufweist, und eine Außenkontur (4) umfasst, die einen Außendurchmesser (D2) im Bereich zwischen 110 mm und 120 mm, vorzugsweise 115 mm, aufweist.

9. Stoma-Set, das mindestens einen Bauchgurt (100) und eine medizinische Vorrichtung (1) nach einem der vorstehenden Ansprüche umfasst.

10. Stoma-Set nach Anspruch 9, das Befestigungsmittel umfasst, die dafür ausgebildet sind, die medizinische Vorrichtung (1) mit dem Umfangsrand (101) der Öffnung (102) am Bauchgurt (100) zu befestigen, wobei die Befestigungsmittel im Bereich des mindestens einen Durchbruchs (8) an der medizinischen Vorrichtung (1) angebracht sind.

11. Stoma-Set nach Anspruch 10, wobei die Befestigungsmittel vom Typ Naht, Nietteil oder Druckteil sind.

## Claims

1. Medical device (1) for ostomy, comprising a ring (2) including an internal contour (3), an external contour (4) and two faces (5, 6), the internal contour defining a central opening (9) configured for receiving a collection appliance (200) for ostomy, said ring comprising a groove (7) extending radially from the external contour thereof, said groove being configured to receive a peripheral edge (101) of an opening (102) used on an abdominal belt (100), **characterised in that** the ring (2) comprises at least one aperture (8) emerging on the two faces (5, 6), said at least one aperture passing through the groove (7).

2. Medical device (1) according to claim 1, wherein the ring (2) is flat.

3. Medical device (1) according to either of the preceding claims, which is produced from a material offering flexibility.

4. Medical device (1) according to claim 3, which is produced from medical silicone.

5. Medical device (1) according to any of the preceding claims, wherein four apertures (8) are uniformly distributed around the ring (2).

6. Medical device (1) according to any of the preceding claims, wherein the groove (7) comprises a width (L2) of between 1.5 mm and 2.5 mm, preferably 2 mm.

7. Medical device (1) according to any of the preceding claims, wherein the groove (2) comprises a depth (L1) of between 18 mm and 23 mm, preferably 21.5 mm.

8. Medical device (1) according to any of the preceding claims, wherein the ring (2) comprises an internal contour (3) having an inside diameter (D1) of between 60 mm and 70 mm, preferably 65 mm, and an external contour (4) having an outside diameter (D2) of between 110 mm and 120 mm, preferably 115 mm.

9. Ostomy kit comprising at least one abdominal belt (100) and a medical device (1) according to any of the preceding claims.

10. Ostomy kit according to claim 9, which comprises fixing means configured to fix the medical device (1) with the peripheral edge (101) of the opening (102) on the abdominal belt (100), said fixing means being fitted at the at least one aperture (8) on said medical device (1).

11. Ostomy kit according to claim 10, wherein the fixing means are of the seam, riveting piece or pressure piece type.
